# EUROPEAN PATENT APPLICATION

(11) **EP 1 698 235 A1**
(43) Date of publication of application: **06.09.2006**
(21) Application number: 04807426.4
(22) Date of filing: 21.12.2004
(51) Int. Cl.: A23L 1/10

(54) **FUNCTIONAL COMPONENT-ENRICHED BARLEY MALT ROOTLETS AND PROCESS FOR PRODUCING THE SAME**

(30) Priority: 22.12.2003 JP 2003424745
(71) Applicant: Sapporo Breweries Limited, Tokyo 150-8522 (JP)
(72) Inventor: Kihara, Makoto, Sapporo Breweries Limited, Gunma, 3700393 (JP); Okada, Yoshihiro, Sapporo Breweries Limited, Gunma, 3700393 (JP); Ito, Kazutoshi, Sapporo Breweries Limited, Gunma, 3700393 (JP)
(74) Representative: Billington, Lawrence Emlyn
(86) International application number: PCT/JP2004/019069
(87) International publication number: WO 2005/065465

(57) **Abstract**

A processing method is disclosed that provides malt root of wheat, barley, oats and rye including lots of functional ingredients such as free amino acids or dietary fibers.

In more detail, the processing methods provide food or food products, medical products and cosmetic products having high functionality, in which products are provided by processing malt root or extracting functional ingredients from malt root, then using the processed malt or the extracted products as a part of manufacturing materials of food or food products, medical products and cosmetic products.

Functional ingredients such as free amino acids like GABA or dietary fibers like β-glucan can be increased by controlling the germination time appropriately, drying or roast-dry temperature appropriately, and both of time of germination and drying or roast-dry temperatures appropriately during soaking, germination and roast-dry processes of malt. Also, extracts of functional ingredients such as free amino acids like GABA or dietary fibers like β-glucan in the extract solution can be increased by optimizing the extraction temperature of malt root in the extract solvent. Therefore, using malt root and its extract obtained by performing the above controls as a part of manufacturing materials, food or food products, medical products and cosmetic products including lots of functional ingredients, and their processing methods can be provided.

## Description

### BACKGROUND OF THE INVENTION

### 1. Field of the Invention

This invention generally relates to malt root of wheat, barley, oats or rye including high functional ingredients contents and their processing method.

### 2. Description of the Related Art

Conventionally, amino acids and dietary fibers are called functional ingredients and they are used as food additives for food processing. It is reported that amino acids have various functions and an improvement of functions and quality of food is possible by using them as food additives within food products. For example, valine is used for improving the flavor of various food products. Also, recently, gamma-aminobutyric acid called GABA is used. It is widely found in nature and it is one type of amino acid; its chemical formula is NH₂CH₂CH₂CH₂COOH. It is known that GABA acts as a neurotransmitter of a restraint system in the human body. In addition, it is known that GABA acts in depressing blood pressure, stabilizing the mind of human beings, improving kidney and liver functions, and promoting alcohol metabolism.

Also, in the cosmetics field, products including natural moisture materials or collagen are becoming very popular. Natural moisture materials are presented in the stratum corneum of the epidermis, and they have an important function to maintain moisture in skin; 40% of their materials are comprised of aspartic acid, alanine, arginine, glutamic acid, threonine, serine, tyrosine, tryptophan, valine, phenylalanine, methionine, lysine, glycine and betaine. Also, these amino acids have high affinity for and promote metabolism of skin cells and moisturize them, and have a function for assisting re-synthesis of collagen.

Dietary fibers have various functions like operations to keep the bowels well-conditioned and restraint operations against high sugar levels in blood. So, water products including dietary fibers are manufactured.

It is well-known by persons who are practiced in the art that processed products of grains, which are germinated grains such as germinated unpolished rice as a food material, include lots of these functional ingredients for richness. For example, see patent documents 1 and 2.

Concerning wheat, barley, oats and rye among the grains, it is well-known by persons who are practiced in the art that as food material including functional ingredients, germinated wheat contains amino acids as functional ingredients, oats contains β-glucan as a dietary fiber, and barley contains functional ingredients of free amino acids and dietary fibers (β-glucan)

On the other hand, malt root has been removed during the malt manufacturing process because malt root gives beer and low-malt beer added flavor in the course of the brewing.

However, according to research of the present inventors, it was recognized that functional ingredients contents like amino acids and dietary fibers are more prevalent in malt root.
Patent document 1:JP2003-250512
Patent document 2:JP2003-159017
Non-patent document 1: Briggs, Malt and Malting, 1998, p.9

### SUMMARY OF THE INVENTION

It is a general object of the present invention to provide food or food products, medical products and cosmetic products having high functionality, in which the above products are provided by processing malt root or extracting functional ingredients from malt root, then using the processed malt root or the extracted products as a part of manufacturing materials of food or food products, medical products and cosmetic products, and their manufacturing process.

In order to achieve the above-mentioned object, as described in claim 1, there is provided a method of processing malt root,
characterized in that any functional ingredients contents in malt root are adjusted by controlling a germination time of malt manufactured through a germination process, after a soaking process for seeds of one or more of wheat, barley, oats and rye.

According to claim 1, by controlling the germination time of malt, the method of processing malt root, in which method any functional ingredients contents such as free amino acids like GABA or dietary fibers contents are adjusted, can be provided. Thereby, malt root with increased functional ingredients contents can be provided.

In order to achieve the above-mentioned object, as described in claim 2, there is provided a method of processing malt root,
characterized in that any functional ingredients contents in malt root are adjusted during the drying process or the roast-dry process at a predetermined temperature, after a process of soaking seeds of one or more of wheat, barley, oats and rye,
wherein a temperature of the drying process or a temperature of the roast-dry process is controlled.

According to claim 2, by controlling the temperature of the drying process or the temperature of roast-dry process after the soaking process for seeds of one or more of wheat, barley, oats and rye, the method of processing malt root, in which any functional ingredients such as free amino acids like GABA or dietary fibers contents are adjusted, can be provided. Thereby, malt root with increased functional ingredients contents can be provided.

In order to achieve the above-mentioned object, as described in claim 3, there is provided a method of processing malt root,
characterized in that any functional ingredients contents in malt root are adjusted during the drying process or the roast-dry process at a predetermined temperature, after the seed soaking process of one or more of wheat, barley, oats and rye,
wherein the germination time, and the temperature of the drying process or the temperature of the roast-dry process are controlled.

According to claim 3, by controlling the germination time, and the temperature of the drying process or the temperature of the roast-dry process during the drying process or the roast-dry process, respectively, at a predetermined temperature, after the soaking process of seeds of one or more of wheat, barley, oats and rye, the method of processing malt root, in which any functional ingredients such as free amino acids like GABA or dietary fibers contents are adjusted, can be provided. Thereby, malt root with increased functional ingredients contents can be provided.

In order to achieve the above-mentioned object, as described in claim 4, there is provided a method of extracting an extract solution of extracted functional ingredients in a malt root, comprising
soaking seeds of one or more of wheat, barley, oats and rye, and
soaking the malt root manufactured by germination in a extract solvent
characterized in that any functional ingredients contents in malt root are adjusted by controlling a temperature of the extract solvent.

According to claim 4, by controlling the temperature of the extract solvent of malt root, the method of extracting the extract solution, by which control of the temperature any functional ingredients such as free amino acids like GABA or dietary fibers contents are adjusted, can be provided. Thereby, extracts from malt root with increased functional ingredients contents can be provided.

In order to achieve the above-mentioned object, as described in claim 5, there is provided a malt root manufactured with methods as claimed in any one of claims 1-3.

According to claim 5, by using methods as claimed in any one of claims 1-3, malt root, in which any functional ingredients such as free amino acids like GABA or dietary fibers contents in the malt root are adjusted, can be provided.

In order to achieve the above-mentioned object, as described in claim 6, there is provided a processed product or processed products using the malt root as claimed in claim 5 or the extracts obtained by the method of extraction as claimed in claim 4 as materials for the processed product or processed products.

According to claim 6, by using malt root as claimed in claim 5 or the extracts obtained by the method of extraction as claimed in claim 4 as materials, food or food products, medical products and cosmetic products may have high functional ingredients such as free amino acids like GABA or dietary fibers contents.

According the present invention, it is possible to provide food or food products, medical products and cosmetic products having high functionality such as free amino acids like GABA or dietary fibers like β-glucan, in which the above products are provided by processing malt root or extracting functional ingredients from malt root, then using the processed malt or the extracted products as a part of manufacturing materials of food or food products, medical products and cosmetic products, and their manufacturing process.

Other objects, features and advantages of the present invention will become more apparent from the following detailed description.

### BRIEF DESCRIPTION OF THE DRAWINGS

Fig. 1A is a graph showing a change of GABA or other free amino acids contents during the germination process of malt root;
Fig. 1B is a graph showing a change of GABA or other free amino acids contents during the germination process of malt root;
Fig. 2 is a graph showing a change of GABA content during the germination process of malt root and malt;
Fig. 3 is a graph showing changes of free amino acids contents at different roast-dry temperatures;
Fig. 4 is a graph showing changes of the GABA content of malt root and malt at different roast-dry temperatures;
Fig. 5 is a graph showing changes of free amino acids contents at different extraction temperatures from malt root;
Fig. 6 is a graph showing changes of the GABA content at different extraction temperatures from malt root;
Fig. 7 is a graph showing changes of free amino acids contents at different extraction temperatures from malt root within 30 minutes;
Fig. 8 is a graph showing changes of free amino acids contents at different extraction temperatures from malt root within 60 minutes;
Fig. 9 is a graph showing a change of free amino acids contents during the manufacturing process of breads manufactured with bread flour mixed with 0% malt root flour;
Fig. 10 is a graph showing a change of free amino acids contents during the manufacturing process of breads manufactured with bread flour mixed with 10% malt root flour; and
Fig. 11 is a graph showing the β-glucan content in breads manufactured in the fourth embodiment.

### DESCRIPTION OF THE PREFERRED EMBODIMENTS

A description will now be given, with reference to the embodiments according to the present invention.

It is recognized by inventors of the present invention that functional ingredients (such as GABA and other free amino acids or dietary fibers like β-glucan) contents included in malt root are more prevalent, and are increased or decreased depending on germination processing time and/or drying or roast-dry temperatures after germination, so that further appropriate process methods or any degree for processing are needed depending on the targeted functional ingredients since a tendency of change is different for each functional ingredient (that is, functional ingredient A content is increased but functional ingredient B is decreased). Thus, the present invention was established by the inventors of the present invention.

The present invention comprises the following steps. Concerning malt root produced by processing barley malt used as materials for beer and low-malt beer, a step is that functional ingredients contents in malt root are increased by controlling process conditions during the manufacturing process of malt; another step is an effective extraction of functional ingredients contents from malt root; another step is manufacturing various food or food products including malt root or its extract; and another step is that functional ingredients contents in food or food products are increased by controlling the manufacturing process or the manufacturing conditions during manufacturing of food or food products using malt root and its extract as materials for food or food products. In the below description, each step is described in more detail. In the experiments of the below description, barley seed (type: Haruna Nijo) was used.

First, a malt processing method and its processing conditions for a manufacturing step of barley malt as a material for beer and low-malt beer (hereinafter called soaking, germination, and roast-dry processes) were controlled, and functional ingredients contents in malt root were increased.

In general, soaking, germination, and roast-dry processes comprise a soaking process, a germination process and a roast-dry process. In detail, barley is soaked in water or hot water and water is absorbed (soaking process); next, barley germ is grown to a length about 2/3 of the whole length of grain particles (germination), and after allowing biosynthesis of an enzyme and decomposition of a part of a stored substance during the above process, the resulting products are heat-dried (dry roasted) to allow for producing malt. Malt root is root which is produced from seeds during the above manufacturing process.

In order to increase functional ingredients contents included in malt root, these functional ingredients contents in seeds and malt root during the soaking, germination, and roast-dry processes were measured with the progression of time. After soaking, the sampling of malt root was performed every day from germination day 1 to day 6, and obtained samples were freeze-dried. Then, samples were made into flour. The powdered sample of 50 mg/800 µl water was shaken overnight at 5 °C, then each content was measured with an amino acids analyzer (Nihon-Denshi).

As a result of the measurement, for free amino acids like proline, their contents were drastically increased after the germination process. It was recognized that most of amino acids contents in malt root were increased greater than those in seeds. Also, most of amino acids including GABA contents showed the highest values at the germination day 1. Thus, it was recognized that GABA and other free amino acids contents in malt root can be adjusted by controlling the germination process of manufacturing for malt root depending on their targeted free amino acids.

Also, the above ingredients contents were measured by changing the roast-dry temperatures. After germination, samples were roast-dried at 37 °C, 45 °C, 55 °C and 85 °C, then relationships between the GABA and other free amino acids contents and the roast-dry temperatures were measured. GABA and other free amino acids contents were measured using the same method as described above. Then, samples were made into flour. The powdered sample of 50 mg/800 µl water was shaken overnight at 5 °C, then each content was measured with an amino acids analyzer (Nihon-Denshi).

As a result of the measurement, the freeze-dried sample showed the highest value of content, also lots of amino acids were increased by roast-drying in a range of 42 °C-55 °C, which is lower than usual roast-dry temperatures. For GABA, similarly, the GABA content can be adjusted by controlling the roast-dry temperature. Thus, GABA and other free amino acids contents in malt root can be adjusted by controlling the manufacturing process (the roast-dry temperature) of malt root depending on their targeted free amino acids. Therefore, it was recognized that these ingredients contents are drastically changed by controlling the soaking, germination, and roast-dry processes.

Next, in order to increase amino acids including GABA contents, effective extraction conditions of amino acids from malt root were examined. Malt from Myogi Nijo and malt root of 50 mg/400 µl water was shaken 30 minutes at different temperatures (25 °C-55 °C), then free amino acids contents in the extraction solution were measured. As a result, it was recognized that extraction of all amino acids except arginine at 45 °C-55 °C is preferable, and the extraction for GABA at 45 °C is most preferable. Thus, GABA and other free amino acids contents in the extract can be adjusted by controlling the extraction temperature of amino acids from malt root depending on their targeted free amino acids, and effective conditions of extracts from malt root for adding to food products were clearly specified.

Next, crushed flour of malt root manufactured with the above method and their extracts were mixed in materials, then bread, pasta, udon, soba and cookies were manufactured with conventional methods for each respective food product. For bread, the mixed ratio of crushed flour or extracts was increased more than the conventional mixed ratio of malt (0.09-0.36%). Also, for the rest of the food products, food products were manufactured with 20-50% mixed ratio of crushed malt flour. For any sample, it was possible for each food sample to have its own characteristics, and it was recognized that food products including malt flour as food material can be manufactured.

Also, the above food products were manufactured by mixing malt root and its extract into the above food products, then GABA and other free amino acids contents in food products were measured. As a result, it was recognized that as the mixed ratio of malt root is increased, these ingredients contents are increased.

Also, food products were manufactured by mixing malt root and its extract thereto, then changes of GABA and other free amino acids contents during the manufacturing process for food products were measured. As the mixed ratio of malt root was increased, GABA and some free amino acids contents were increased during their manufacturing processes by controlling temperatures for the fermentation step, for example, for fermented food products.

Therefore, GABA and other free amino acids contents in malt root can be increased by appropriately controlling the germination period and the drying or roast-drying temperature during the soaking, germination, and roast-dry processes of malt. Moreover, GABA and other free amino acids contents in the extract from malt root can be increased by optimizing extraction conditions of the malt root.

According to the present invention, malt root and its extract obtained by controlling the above conditions are used as food materials, then food products with increased GABA and targeted free amino acids contents can be manufactured.

In the below description, embodiments of the present invention are described in more detail.

### First Embodiment

The measurement with progression of time of the GABA and other free amino acids contents in the germination step during the soaking, germination and roast-dry processes of malt

In order to increase the GABA and other free amino acids contents in food products with malt root as a food material, these ingredients contents in seeds, malt and malt root during soaking, germination and roast-dry processes (Haruna Nijo) were measured with the progression of time. Soaking, germination and roast-dry processes of pilot samples were in accordance with standard methods of the Sapporo Beer Corporation. After soaking, sampling was performed every day from germination day 1 to day 6, then the obtained samples were freeze-dried. GABA and other free amino acids contents were measured as following. Samples were made into flour. The powdered sample of 50 mg/800 µl water was shaken overnight at 5 °C, then each content was measured with an amino acids analyzer (Nihon-Denshi).

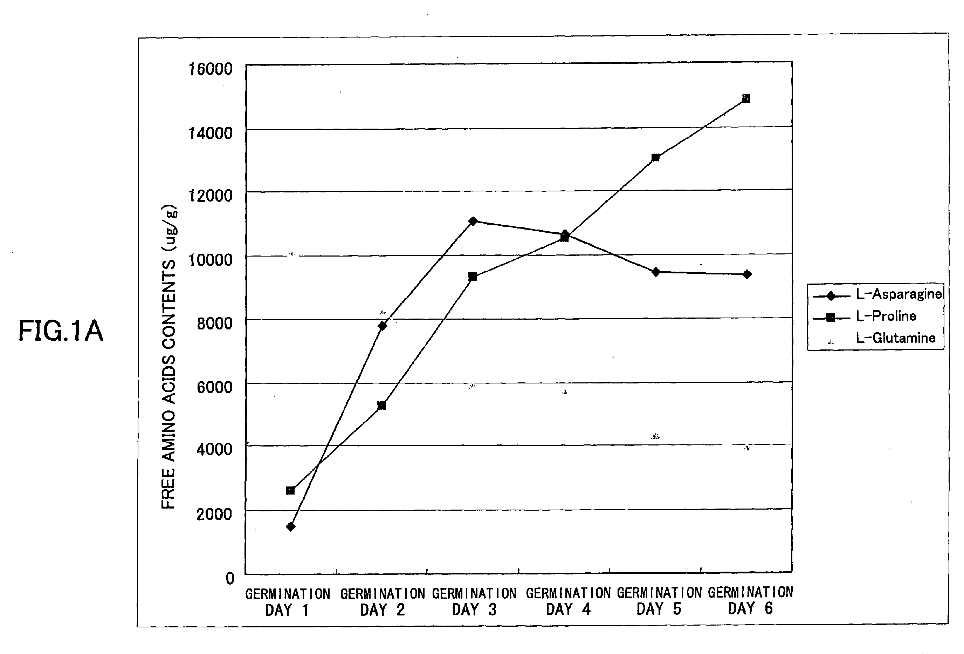

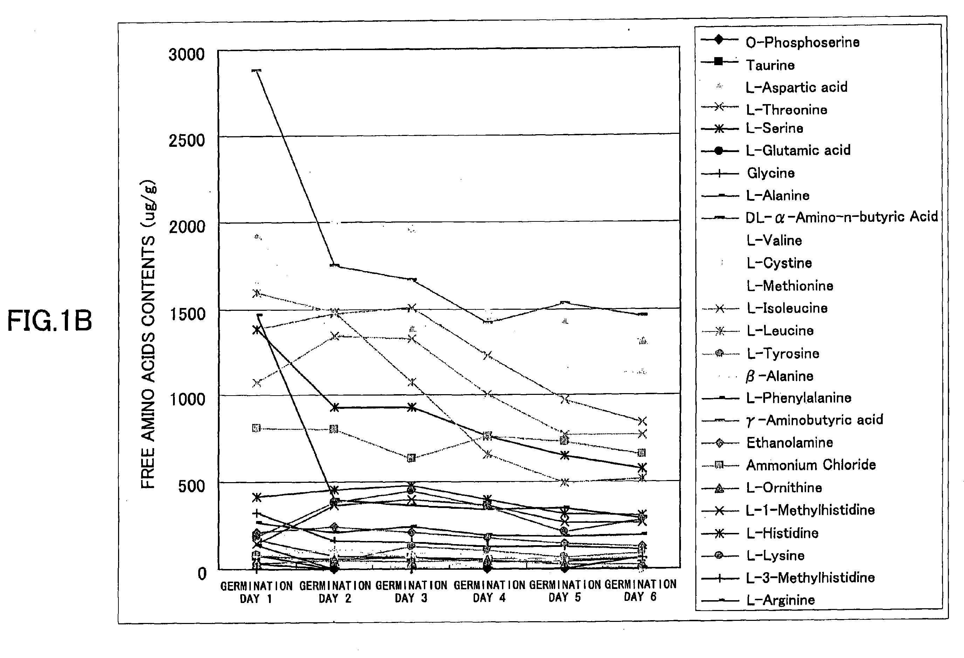

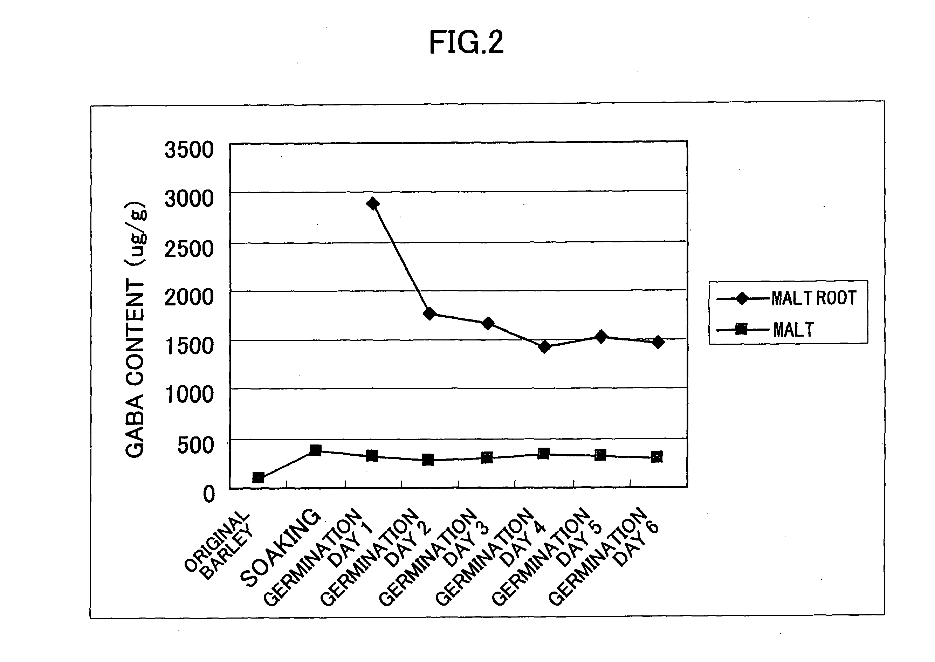

As a result of the measurement, for free amino acids like proline, their contents were drastically increased after the germination process. Most of amino acids contents in malt root were increased 5-10 times those in malt. It was recognized that most amino acids including GABA contents showed the highest values at the germination day 1, according to the result. Figure 1 shows a change of GABA or other free amino acids contents during the germination process of malt root. Figure 2 shows a change of GABA content during the germination process of malt root and malt. Thus, GABA and other amino acids contents in malt root can be adjusted depending on its purpose by controlling the germination period of time during the germination process of malt.

### Second embodiment

The measurement of the GABA and other free amino acids contents at roast-dry temperature during soaking, germination and roast-dry processes

In order to increase the GABA and other free amino acids contents in food products with malt root as a food material, after germination, samples were roast-dried at 37 °C, 45 °C, 55 °C and 85 °C, then relationships between the GABA and other free amino acids contents and the roast-dry temperatures were measured. GABA and other free amino acids contents were measured by the same method as described in the first embodiment.

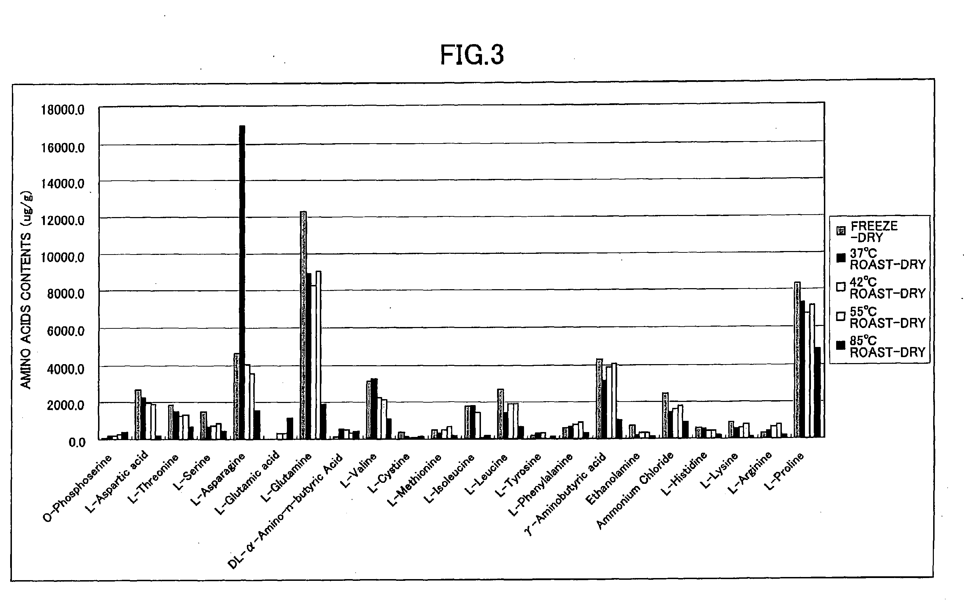

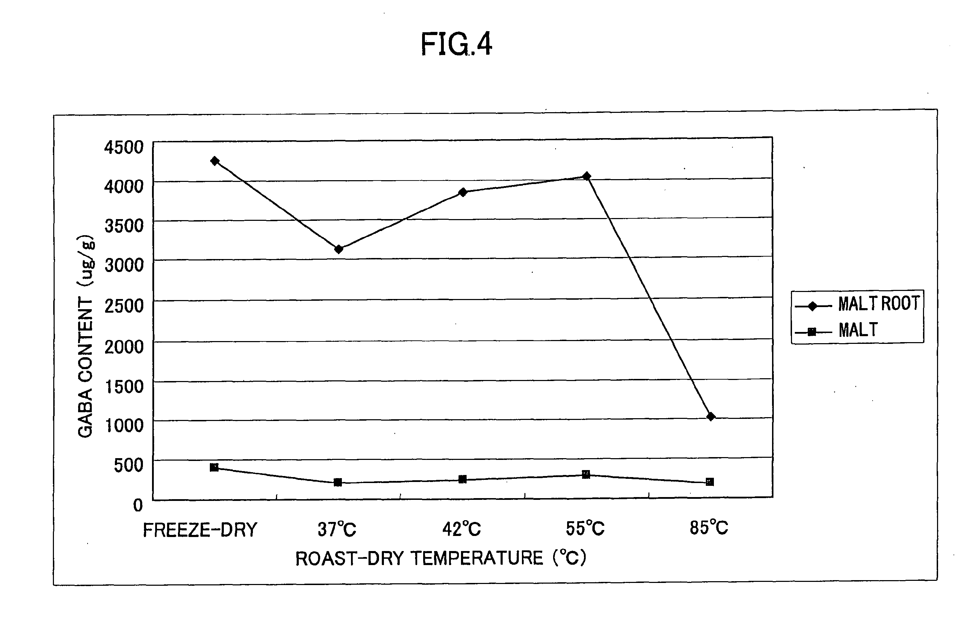

As a result of the measurements, the freeze-dried sample showed the highest value of content; also lots of amino acids were increased by roast-drying in a range of 42 °C-55 °C, which is lower than usual roast-dry temperatures. Figure 3 shows changes of free amino acids contents for different roast-dry temperatures. For GABA, similarly, the GABA content could be adjusted by controlling the roast-dry temperature.(Figure 4) Figure 4 shows a change of the GABA content of malt root and malt in a difference of roast-dry temperature. Thus, GABA and other free amino acids contents in malt root can be adjusted by controlling the manufacturing process (the roast-dry temperature) of malt root depending on their targeted free amino acids. However, for GABA, it is not realistic to use an industrial method where lots of germinated malt, which is made by soaking barley, is freeze-dried. Thus, it is appropriate that a drying process, accomplished by a heating process for which the highest value was shown at 55 °C, be performed.

### Third embodiment

Examination of effective extraction conditions of amino acids from malt root

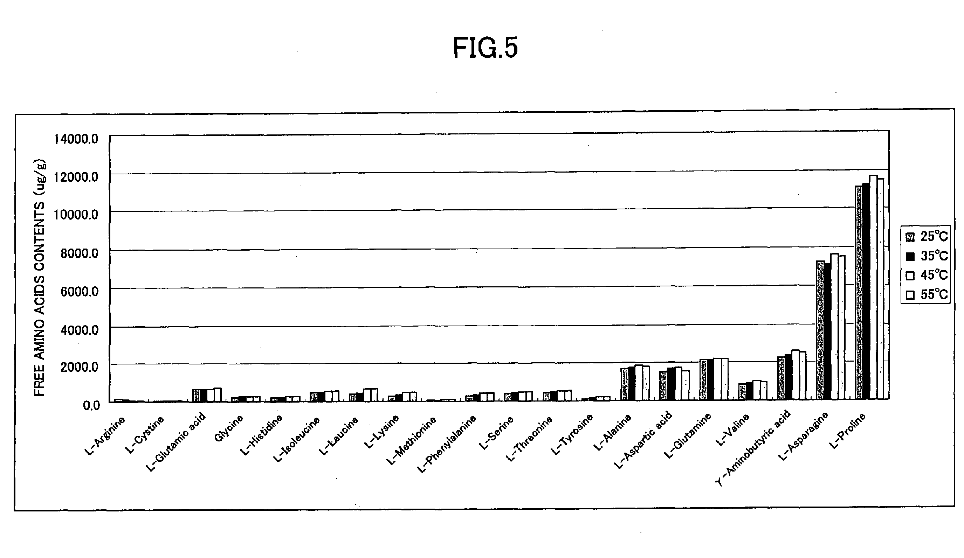

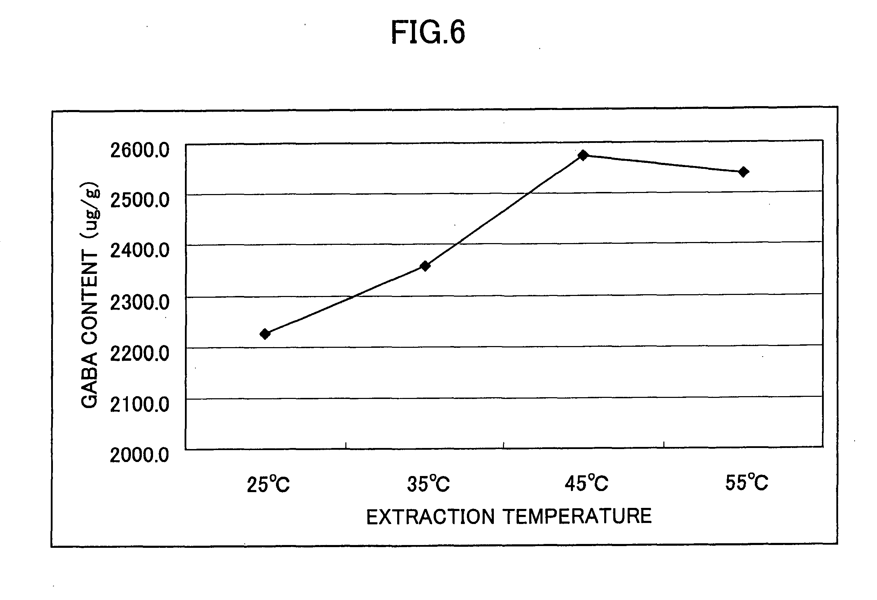

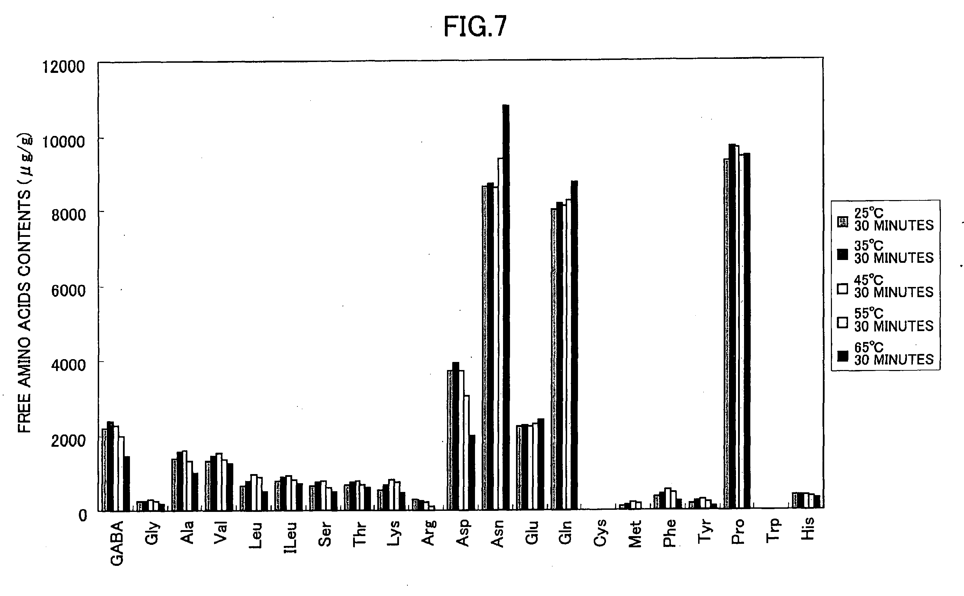

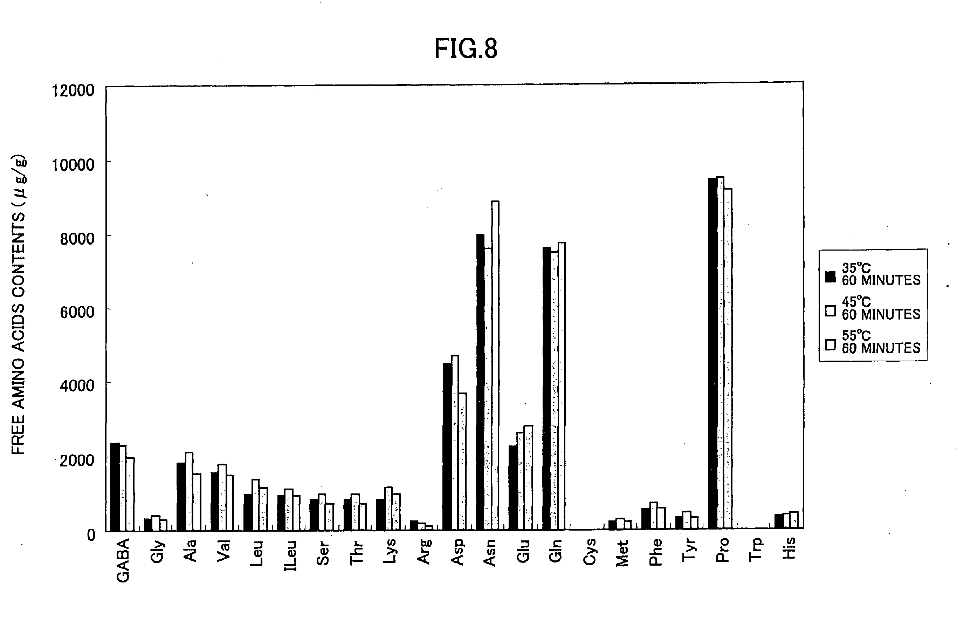

In order to increase amino acids including GABA contents, effective extraction conditions of amino acids from malt root were examined with malt root extracts as food materials. Malt from Myogi Nijo and malt root of 50 mg/400 µl water was shaken 30 minutes at different temperatures (25 °C-55 °C), then free amino acids contents in the extraction solution were measured. As a result, it was recognized that extraction of all amino acids except arginine at 45 °C-55 °C is preferable. Figure 5 shows changes of free amino acids contents for different extraction temperatures for malt root. Also, it was recognized that the extraction for GABA at 45 °C is most preferable. Figure 6 shows changes of the GABA content at different extraction temperatures for malt root. Similarly, extraction temperature and extraction time were examined using different barley (Ryohu). (figures 7 and 8) As a result, the highest values for GABA and aspartic acid occurred at 35 °C extraction temperature and the highest values for asparagine and glutamine occurred at 65 °C extraction temperature. Concerning the influences of the extraction time, it was recognized that as the extraction time is increased, most free amino acids contents were not changed or were a little bit increased; on the other hand, asparagine and glutamine contents were decreased. Thus, it was recognized that GABA and other free amino acids contents in the extraction of amino acids from malt root can be adjusted by controlling the extraction temperature depending on their targeted free amino acids.

### Fourth embodiment

A change of free amino acids contents during bread manufacturing process with malt root flour as material for breads

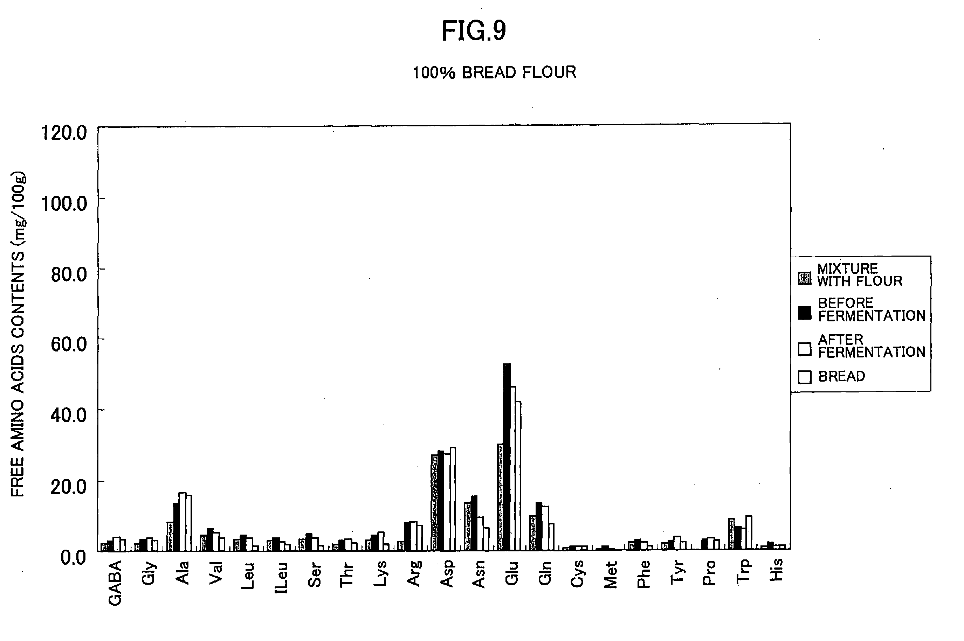

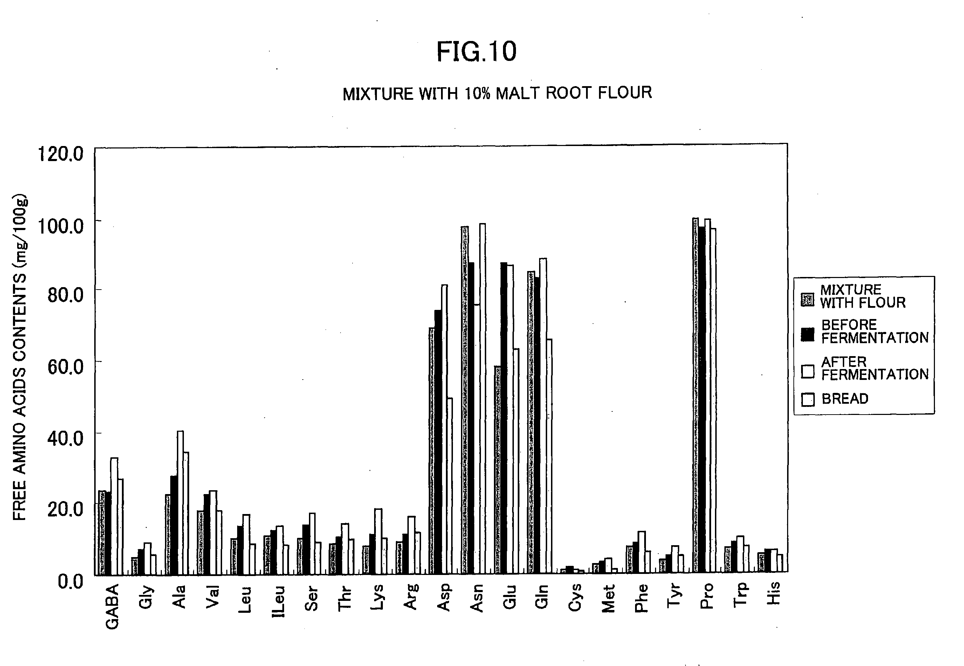

Malt root flour was produced by crushing into powder malt root germinated in day 6 during the germination process of malt (Haruna Nijo) as in the first embodiment. Roll Breads were manufactured with the conventional manufacturing process of breads by mixing 0% and 10% of the above malt root flour with bread flour. Breads were manufactured based on these materials, and the change of GABA and other free amino acids contents during breads manufacturing processes were measured (figures 9 and 10).

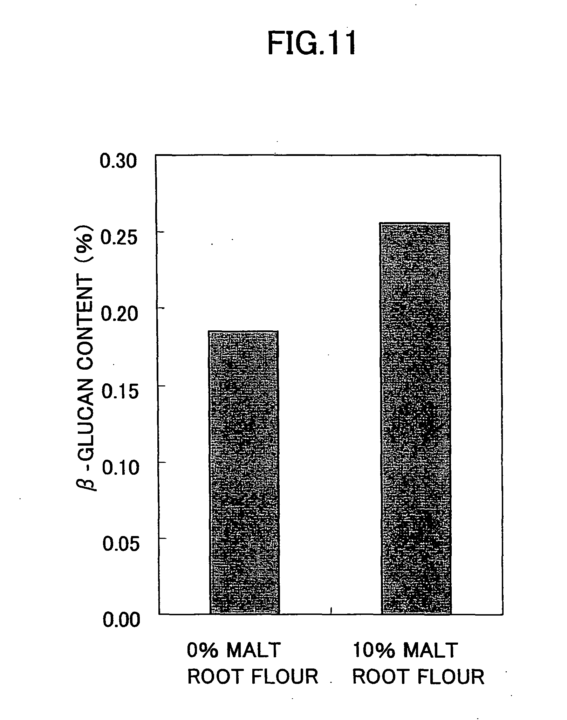

Based on a result of analysis, as malt root flour was mixed, free amino acids contents were also increased. However, it is recognized that functional ingredients contents were increased for some free amino acids like GABA during the fermentation process. Further, the β-glucan content in breads was measured (see figure 11). It was recognized that the β-glucan content in breads was increased by mixing malt root flour, as well as free amino acids.

### Fifth embodiment

A change of the GABA content during pasta manufacturing process

Malt root flour was produced by crushing into powder malt root germinated in day 6 during the germination process of malt (Haruna Nijo) as in the first embodiment. Pasta was manufactured with the conventional manufacturing process of pasta by mixing the above malt root flour. Then, the change of GABA content during the aging process of dough, i.e. the fermentation process, was measured. Also, instead of using malt root flour for a part of pasta materials, malt root extracts manufactured in the first embodiment may be used. Although the temperature for the aging process is ambient temperature for the conventional method, the temperature for the aging process was 45 °C for the fifth embodiment. In this embodiment, 40% bread flour, 40% pastry flour and 20% malt root flour was the mixed ratio, for example. As a result, it was recognized that the GABA content in pasta dough was increased by mixing malt root flour and performing the aging process.

### Sixth embodiment

Manufacturing of udon and soba mixed with malt root and its extract in their materials

Malt root flour was manufactured by crushing into powder malt root, normally made through soaking, germination and roast-dry processes, with only the soaking process. Using the above malt root flour, udon was produced with a conventional method. Comparison examples were performed using 0% and 20% malt root flour mixing ratio to all purpose flour. In the case of 0% and 20% malt root flour mixing ratio, there were no different characteristics between them other than outer appearance of color. Also, in the case of 50% malt root flour mixing ratio, there were the same characteristics as udon with 20% malt root flour mixing ratio.

Thus, udon which includes lots of GABA and other free amino acids can be manufactured.

Comparison examples were performed for soba. Soba was produced using 60% soba flour and 40% all purpose flour, and 60% soba flour and 20% all purpose flour and 20% malt root flour. There were no different characteristics between them, and the characteristics of soba with 20% malt root flour were the same as the characteristics of soba.

Also, a soba-like noodle as new noodles which has 50% all purpose flour and 50% malt root flour was produced with the conventional soba method. As a result, soba-like noodles which have no soba flour and the same appearance and color as soba could be produced. There is no need to worry about soba allergy because the soba-like noodles do not use soba flour. Also, the soba-like noodles include lots of GABA and other free amino acids.

### Seventh embodiment

Manufacturing of fried foods using malt root flour

By using malt root flour made from malt with the germination day controlled or malt with only the soaking process, by mixing 50% of the above molt root flour with fried chicken flour, fried chicken was cooked with the conventional method, and it was compared with fried chicken cooked with 100% fried chicken flour. As a result of taste sampling, because fried chicken cooked with malt root flour includes lots of amino acids as ingredients for taste, fried chicken cooked with malt root flour tasted good. Also, the above malt root flour includes protease activity. Thereby, in mixing the above malt root flour with fried chicken flour, it was expected that fried chicken would have included lots of GABA and other free amino acids; also a feeling of appetite of the fried chicken being soft due to the protease can be provided.

The present invention is not limited to the specifically disclosed embodiments, and variations and modifications may be made without departing from the scope of the present invention.

The present application is based on Japanese Priority Application No.2003-424745 filed on December 22, 2003, the entire contents of which are hereby incorporated by reference.

## Claims

1. A method of processing malt root,
**characterized in that** any functional ingredients contents in the malt root are adjusted by controlling a germination time of the malt root manufactured through a germination process, after a soaking process for seeds of one or more of wheat, barley, oats and rye.

2. A method of processing malt root,
**characterized in that** any functional ingredients contents in the malt root are adjusted during a drying process or a roast-dry process at a predetermined temperature, after a soaking process of seeds of one or more of wheat, barley, oats and rye,
wherein the temperature of the drying process or the temperature of the roast-dry process is controlled.

3. A method of processing malt root,
**characterized in that** any functional ingredients contents in the malt root are adjusted during a drying process or a roast-dry process at a predetermined temperature, after a soaking process of seeds of one or more of wheat, barley, oats and rye,
wherein the germination time, and the temperature of the drying process or the temperature of the roast-dry process are controlled.

4. A method of extracting an extract solution of functional ingredients in a malt root, comprising the steps of
soaking seeds of one or more of wheat, barley, oats and rye, and
soaking the malt root manufactured by germination in an extract solvent;
**characterized in that** any functional ingredients contents in the malt root are adjusted by controlling a temperature of the extract solvent.

5. A malt root manufactured with methods as claimed in any one of claims 1-3.

6. A processed product or processed products using the malt root as claimed in claim 5 or the extracts obtained by the method of extraction as claimed in claim 4 as materials for the processed product or processed products.
